# EUROPEAN PATENT APPLICATION

(11) **EP 4 047 090 A1**
(43) Date of publication of application: **24.08.2022**
(21) Application number: 20875821.9
(22) Date of filing: 16.10.2020
(51) Int. Cl.: C12N 15/09, C07K 14/435, C12N 9/12

(54) **METHOD FOR PREPARING CELL-FREE PROTEIN EXPRESSION GEL USING PLASMID**

(30) Priority: 18.10.2019 KR 20190130097
(71) Applicant: H&Bio Co., Ltd., Seongnam-si, Gyeonggi-do 13605 (KR)
(72) Inventor: LEE, Jong Bum, Seoul 05555 (KR); NAM, Hyang Su, Seoul 02492 (KR)
(74) Representative: dompatent von Kreisler Selting Werner - Partnerschaft von Patent- und Rechtsanwälten mbB
(86) International application number: PCT/KR2020/014157
(87) International publication number: WO 2021/075902

(57) **Abstract**

The present invention relates to a method for preparing a cell-free protein expression gel and, more specifically, to a method for preparing a cell-free protein expression gel, wherein a double-strand circular plasmid including a base sequence containing genetic information for expressing a target protein is separated into single-strand circular plasmids, and elongated DNA strands obtained through DNA polymerase chain reaction are complementarily bound to each other, thereby preparing a cell-free protein expression gel.

## Description

### Technical Field

The present invention relates to a method for producing a cell-free protein expression gel and. More specifically, the present invention relates to a method for preparing a cell-free protein expression gel, the method being capable of forming a cell-free protein expression gel by separating a circular double-stranded plasmid containing a nucleotide sequence containing genetic information for the expression of a target protein into circular single-stranded plasmids, and complementarily binding (i.e., hybridizing) long DNA strands obtained through a DNA polymerase reaction.

### Background Art

Proteins account for the second highest percentage of the human body while water accounts for the highest percentage. Proteins constitute and regenerate the muscles and most of the bones in the body. In addition, proteins perform numerous roles, such as forming antibodies in the immune system to defend against virus and participating in balancing all parts of the body. Therefore, various studies on protein production have been conducted from the past. As a method for producing proteins, as described in the patent document described below, a method of recombining proteins expressed through genetic recombination in living cells is common.

### <Patent Document>

Korean Patent No. 10-1173272 (registered as of August 6, 2012) "Protein Production Method Using Auto Inducible Expression Promoter and Mutant"

However, the method takes a long time because a gene must be newly introduced, the optimal strain must then be determined, the strain must be cultured again, and cells must be disrupted, separated, and purified whenever the type of protein to be produced changes. In addition, since the temperature must be controlled for each process, the reaction time is long. In addition, since cells are used to produce proteins, toxicity problems may occur.

### Technical Problem

The present invention has been devised to solve the aforementioned problems.

One objective of the present invention is to provide a method for preparing a cell-free protein expression gel using a plasmid. The gel is immiscible with a solution so the gel can be easily handled and is easily applicable in vivo. The gel can also increase protein production efficiency and improve stability.

In addition, another objective of the present invention is to provide a method for preparing a cell-free protein expression gel, the method being capable of preparing a cell-free protein expression by simple manipulation using a circular double-stranded plasmid and of improving economic feasibility.

A further objective of the present invention is to provide a method for preparing a cell-free protein expression gel using a plasmid, the method being capable of easily producing various proteins by modifying a nucleotide sequence capable of expressing a specific protein in the plasmid.

In addition, a yet further objective of the present invention is to provide a method for preparing a cell-free protein expression gel using a plasmid so that the gel can be easily changed in shape and volume thereof, can be used in various applications, and can easily control the protein expression concentration and expression time.

### Technical Solution

In order to achieve the aforementioned objectives, the present invention is implemented by embodiments having the configurations described below.

According to one embodiment of the present invention, there is provided a method of preparing a cell-free protein expression gel, the method including: a process of preparing a plasmid that preparing a circular double-stranded plasmid which comprises a nucleotide sequence containing genetic information for expressing a target protein; a process of separating a plasmid that separating the circular double-stranded plasmid into circular single-stranded plasmids; and a process of forming a gel that supplying a DNA polymerase to a container which contains the circular single-stranded plasmids separated from the process of separating a plasmid and then incubating, DNA strands generated from each of the single-stranded plasmids are bound complementally to form the cell-free protein expression gel.

According to another embodiment of the present invention, in the method of preparing a cell-free protein expression gel, the process of separating a plasmid is characterized in that separating the circular double-stranded plasmid into the circular single-stranded plasmid by breaking a hydrogen bond between the double-strand by applying heat to the circular double-stranded plasmids.

According to a further embodiment of the present invention, in the method of preparing a cell-free protein expression gel, wherein through the process of separating plasmid, two circular single-stranded plasmids which are complementary to each other are formed from one circular double-stranded plasmid.

According to a yet further embodiment of the present invention, in the method of preparing a cell-free protein expression gel, the circular double-stranded plasmid comprises a first strand comprising the nucleotide sequence containing genetic information for expressing the target protein, and a second strand having a nucleotide sequence complementary to the first strand, wherein the first strand and the second strand are bonded complementally.

According to a yet further embodiment of the present invention, in the method of preparing a cell-free protein expression gel, the process of forming a gel comprises: a primer binding process that binding a primer with the single-stranded plasmid, wherein the primer has a complementary nucleotide sequence to the circular single-stranded plasmid, in order to repeatedly generate a desired DNA from the separated circular single-stranded plasmid; a polymerase supplying process that supplying the DNA polymerase to the container which contains the circular single-stranded plasmid which are bonded with the primer, after the primer binding process; and an incubating process that treating the container at a certain temperature for a certain time after the polymerase supplying process.

According to a yet further embodiment of the present invention, in the method of preparing a cell-free protein expression gel, two types of primers which have different nucleotide sequences each other are used in the primer binding process.

According to a yet further embodiment of the present invention, in the method of preparing a cell-free protein expression gel, the incubating process is characterized in that long DNA strands are continuously generated from each of the circular single-stranded plasmid, wherein since the long DNA strands have a complementary nucleotide sequence to each other, the long DNA strands are bonded complementally and tangled each other to form the cell-free protein expression gel.

According to a yet further embodiment of the present invention, in the method of preparing a cell-free protein expression gel, the incubating process is characterized in that performed at a temperature in the range of 35°C or more to 39°C or less for 36 hours or more to 96 hours or less.

According to a yet further embodiment of the present invention, in the method of preparing a cell-free protein expression gel, the cell-free protein expression gel is capable of producing the desired protein in a cell-free environment, not in a cell by reacting with a cell lysate.

### Advantageous Effects

The aforementioned embodiments of the present invention provide the advantages described below.

The present invention is in the form of a gel immiscible with a solution, so it is easy to handle and is easily applicable in vivo, and the present invention has the effect of increasing protein production efficiency and improving stability.

In addition, the present invention can prepare a cell-free protein expression gel by simple manipulation using a circular double-stranded plasmid, thereby improving economic efficiency.

In addition, the present invention has the effect of easily producing various proteins by changing the nucleotide sequence capable of expressing a specific target protein in the plasmid.

In addition, the present invention has the effect of easily controlling the shape and volume of the gel, thereby being capable of easily applying the gel in various applications and, and of easily controlling the protein expression concentration and expression time.

### Description of Drawings

FIG. 1 is a diagram for reference to illustrate a method of preparing a cell-free protein expression gel, according to one embodiment of the present invention.
FIG. 2 is a digitalized image obtained after staining the cell-free protein expression gel.
FIG. 3 is a digitalized image obtained after staining the cell-free protein expression gel and then irradiating the cell-free protein expression gel with ultraviolet rays.
FIG. 4 is an atomic force microscope image for investigating a process of forming a cell-free protein expression gel according to a reaction time.
FIG. 5 is a 3D image converted from the image of FIG. 4.
FIG. 6 is an SEM image for investigating a process of forming a cell-free protein expression gel according to a reaction time.
FIG. 7 is a chart showing the measurement results of the viscoelasticity of a cell-free protein expression gel.
FIG. 8 is an image obtained from an electrophoretic image analysis system for investigating protein production of a cell-free protein expression gel.
FIG. 9 is a chart showing results obtained from a real-time polymerase chain reactor for investigating protein production of a cell-free protein expression gel.

### Best Mode

Hereinafter, a method for preparing a cell-free protein expression gel using a plasmid, according to the present invention, will be described in detail with reference to the drawings. Unless otherwise specified, all terms used in the present specification have the same meaning as the general meaning of the terms understood by a person with ordinary skill in the art to which the present invention belongs, and if the general meaning conflicts with the meaning of a term used in the present specification, the definition used in the present specification is applied. Moreover, detailed descriptions of well-known functions and configurations, which may unnecessarily obscure the gist of the present invention, will be omitted. It will be further understood that the terms "comprise", "includes" or "has", when used in this specification, specify the presence of an element, but do not preclude the presence or addition of one or more other elements unless the context clearly indicates otherwise.

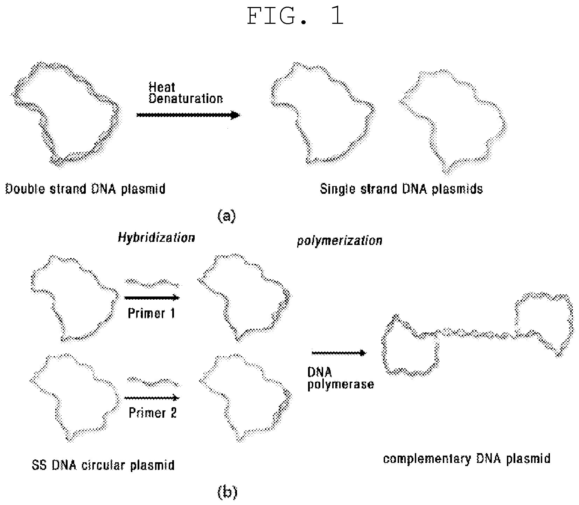

A method for preparing a cell-free protein expression gel using a plasmid, according to one embodiment of the present invention, will be described with reference to FIGS. 1 to 9. The method includes: a plasmid production process of producing a circular double-stranded plasmid containing a nucleotide sequence containing genetic information for expression of a target protein; a plasmid separation process of separating the circular double-stranded plasmid into circular single-stranded plasmids; a cell-free protein expression gel formation process of supplying a DNA polymerase to a container containing the circular single-stranded plasmids separated in the plasmid separation process and of performing incubation, so that DNA strands generated from each circular single-stranded plasmid complementarily bind each other to form the cell-free protein expression gel.

The plasmid production process is a process of preparing a circular double-stranded plasmid containing a nucleotide sequence containing genetic information for expressing a target protein. The circular double-stranded plasmid includes: a first strand containing the nucleotide sequence containing genetic information for expressing the target protein; and a second strand having a nucleotide sequence complementary to the first strand. The first strand and the second strand are bound complementarily. Since the process of producing a circular double-stranded plasmid including a nucleotide sequence containing genetic information for expression of a target protein is an existing art, a detailed description thereof will be omitted.

The plasmid separation process is a process of separating a circular double-stranded plasmid produced through the plasmid production process into circular single-stranded plasmids. In this process, it is possible to separate a circular double-stranded plasmid into circular single-stranded plasmids by breaking the hydrogen bond of the double-stranded plasmid through thermal treatment. Through the plasmid separation process, two circular single-stranded plasmids having nucleotide sequences complementary to each other can be formed from one circular double-stranded plasmid. The plasmid separation process may be performed, for example, by treatment at a temperature in the range of 90°C to 100°C for 3 to 7 minutes.

The gel formation process is performed by supplying a DNA polymerase to a container that contains the circular single-stranded plasmids obtained through the plasmid separation process and then performing incubation. In this process, DNA strands generated from the respective single-stranded plasmids are complementarily bound (i.e., hybridized) to form the cell-free protein expression gel. That is, the gel formation process includes a primer binding process, a polymerase supply process, an incubation process, and the like.

The primer binding process is a process of binding a primer having a nucleotide sequence complementary to that of the circular single-stranded plasmid to the circular single-stranded plasmid to repeatedly produce the desired DNA from the separated circular single-stranded plasmid. In the plasmid separation process, two circular single-stranded plasmids having nucleotide sequences complementary to each other are formed per one circular double-stranded plasmid. Therefore, in the primer binding process, two types of primers having different nucleotide sequences may be used.

The polymerase supply process is a process of supplying a DNA polymerase to a container containing the primer-coupled circular single-stranded plasmids after the primer binding process is performed.

The incubation process is a process of treating the container at a predetermined temperature for a predetermined time after the polymerase supply process. In the incubation process, long DNA strands are continuously generated from each circular single-stranded plasmid, and the long DNA strands are complementary to each other. Therefore, the long DNA strands complementarily bind each other and entangle through complementary binding to form a cell-free protein expression gel. The incubation process may be performed, for example, by treatment at a temperature in the range of 35°C to 39°C for 36 to 96 hours. The cell-free protein expression gel produced by the cell-free protein expression gel preparation method using a plasmid is in the form of a gel that is immiscible with a solution. Therefore, the gel is easy to handle and is easy to apply in vivo. In addition, it is possible to produce the target protein in a cell-free environment rather than in a cell by reacting the cell-free protein expression gel with a cell lysate, resulting in increase in protein production efficiency and improvement in stability. The cell-free protein expression gel preparation method described above can prepare a cell-free protein expression gel in a simple manner by using a plasmid. In addition, the method can easily produce various proteins by changing the nucleotide sequence capable of expressing a specific desired protein in the plasmid. In addition, since the shape and volume of the gel can be easily controlled, the gel can be used in various applications. In addition, the concentration of proteins expressed can be increased, and a sustained expression time can be controlled.

Another embodiment of the present invention relates to a cell-free protein expression gel prepared by the cell-free protein expression gel preparation method using a plasmid.

Hereinafter, the present invention will be described in more detail with reference to examples. However, these examples are presented merely for describing the present invention in more detail, and the scope of the present invention is not limited to these examples.

### <Example 1> Preparation of Cell-Free Protein Expression Gel

1. To easily and quantitatively analyze a protein produced in a prepared gel, a circular double-stranded plasmid including a nucleotide sequence (SEQ ID NO: 1) containing genetic information for expression of a green fluorescent protein was prepared. The circular double-stranded plasmid has a top strand (SEQ ID NO: 1) including a nucleotide sequence containing genetic information for expression of a green fluorescent protein, and a bottom strand (SEQ ID NO: 2) including a nucleotide sequence complementary to that of the top strand (SEQ ID NO: 1). The top strand and the bottom strand are bound to each other.
2. The circular double-stranded plasmid was put into a tube containing nuclease free water (plasmid concentration 30 nM, total volume 20 µl), followed by reaction at 95°C for 5 minutes to obtain two types of circular single-stranded plasmids.
3. Next, primer 1 [5, -AAGTCGTGCTGCTTCATGTG-3, (SEQ ID NO: 3)] complementary to a first type of circular single-stranded plasmid, and primer 2 [ 5'-TAATACGACTCACTATAGGGAT-3' (SEQ ID NO: 4)] complementary to a second type of circular single-stranded plasmid were prepared. Primers 1 and 2 were put into the tube (the concentrations of primers 1 and 2 were each 30 uM, and the total volume was 20 µl). With the use of a thermal cycler, primer 1 was allowed to bind to the first type of circular single-stranded plasmid, and primer 2 2 was allowed to bind to the second type of circular single-stranded plasmid.
4. Phi29 DNA polymerase (6 units), reaction buffer 2µl (400 mM Tris-HCl, 500 mM KC1, 100 mM MgCl₂, 50 mM (NH₄)₂SO₄, 40 mM DTT), and dNTP mix (6.25 mmol) were put into the tube, and then the content in the tube was heated to 37°C, followed by incubation for 72 hours to form a cell-free protein expression gel.

### <Example 2> Identification of Constituent Materials and Morphology of Cell-Free Protein Expression Gel

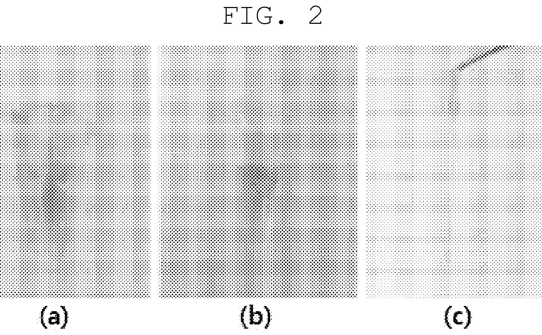

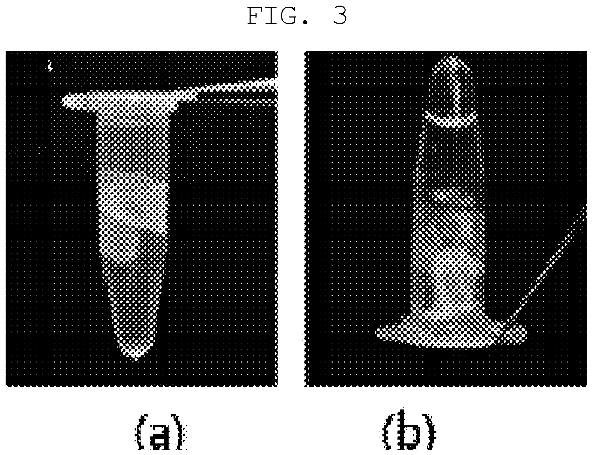

1. Gelred, which is a fluorescent material for staining only nucleic acids, was put into the tube in which the protein expression gel prepared in Example 1 was formed, and the result was photographed with a digital camera. The results are shown in FIG. 2. In FIG. 2, (a) is an image obtained by injecting a fluorescent material into the tube and shooting immediately, (b) is an image obtained in 10 minutes after the injection of the fluorescent material into the tube, and (c) is an image obtained by taking the protein expression gel shown in FIG. 2b with tweezers out of the tube and then photographing the gel.
2. The protein expression gel that was stained with a fluorescent material in stage 1 of Example 2 was put into a new tube, irradiated with UV, and photographed with a digital camera. The results are shown in (a) of FIG. 3. In addition, the tube shown in (a) of FIG. 3 was turned over and was then photographed. The results are shown in (b) of FIG. 3.
3. Comparing (a) and (b) of FIG. 2, it is seen that the fluorescent material penetrates only into the gel as time passes. Therefore, it is seen that the protein expression gel prepared in Example 1 is a nucleic acid-based structure. Referring to (c) of FIG. FIG. 2, the gel has a shape that can be grasped with tweezers. Referring to (b) of FIG. 3, it is confirmed that the protein expression gel prepared in Example 1 has the form of a gel hat is not miscible with a solution even when the tube is turned over. That is, it is seen that the protein expression gel prepared in Example 1 is in the form of gel.

### <Example 3> Investigation of Process of Forming Cell-Free Protein Expression Gel According to Reaction Time

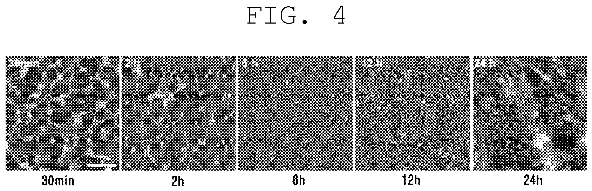

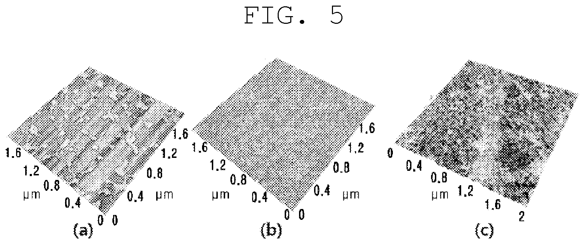

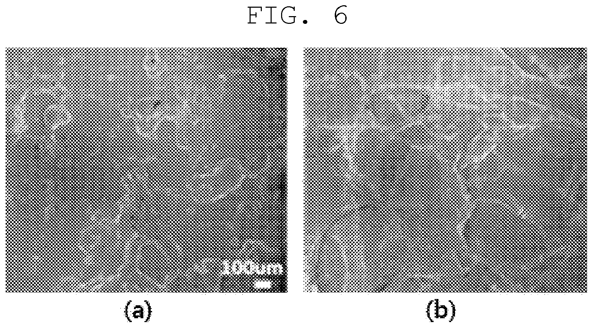

1. The process of forming a gel in stage 4 of Example 1 was investigated over reaction time (for example, 30 minutes, 2 hours, 6 hours, 12 hours, and 24 hours of reaction). The results are shown in FIG. 4. The images obtained at 30 minutes of reaction, 6 hours of reaction, and 24 hours of reaction shown in FIG. 4 were converted into 3D images which are illustrated in FIG. 5 ((a), (b), (c), and (d) in FIG. 5 are 3D images obtained after 30 minutes , 2 hours, 6 hours, 12 hours, and 24 hours of reaction, respectively). In addition, after 48 hours of reaction and after 72 hours of reaction, the protein expression gel was observed with a scanning electron microscope (SEM), and the results are shown in FIG. 6 ((a) FIG. 6 is a SEM image obtained after 48 hours of reaction and (b) of FIG. 6 is an SEM image obtained after 72 hours of reaction).
2. Referring to FIGS. 4 to 6, as the reaction time increases, it is confirmed that the DNA strands having the complementary nucleotide sequences generated through the DNA infinite replication process aggregate in a net form to form a gel.

### <Example 4> Measurement of Viscoelasticity of Cell-Free Protein Expression Gel

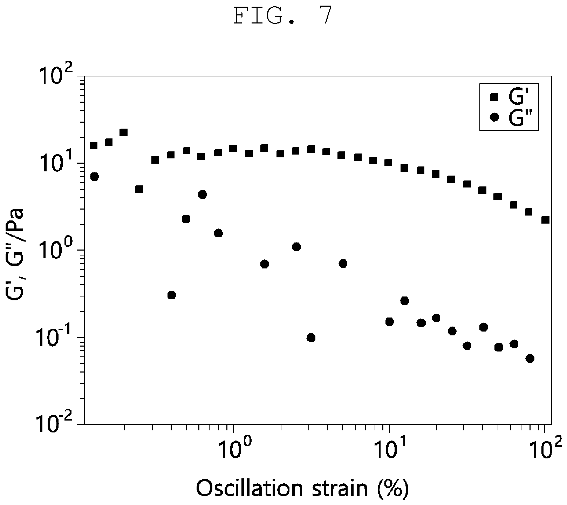

1. The protein expression gel prepared in stage 4 of Example 1 was measured with a rheometer, and the results are shown in FIG. 7.
2. Referring to FIG. 7, it is seen that A G' value is larger than a G" value in the given strain strength range. This indicates that the protein-expressing gel has the characteristics of a gel.

### <Example 5> Observation of Protein Production in Cell-Free Protein Expression Gel

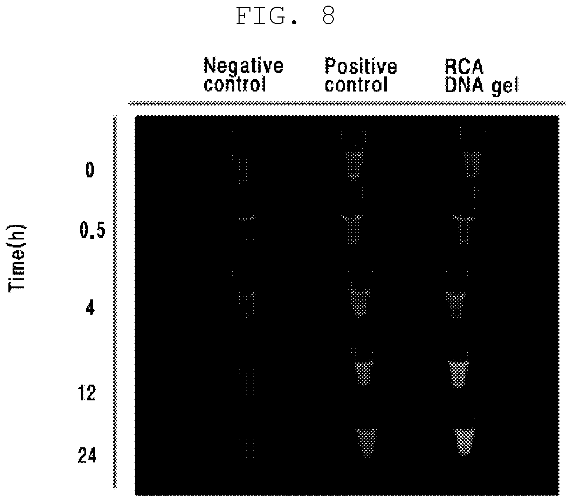

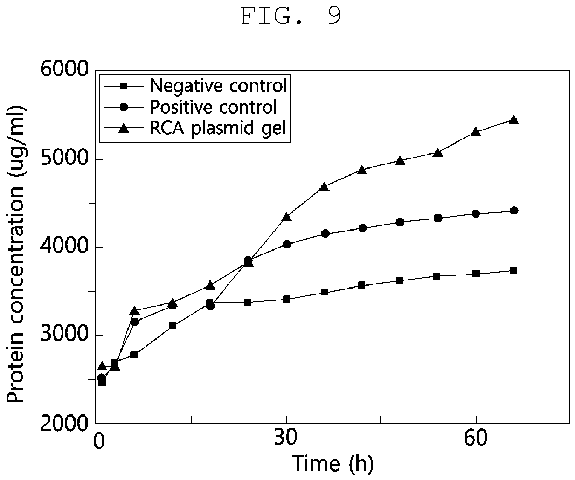

1. A cell lysate (Hela cell lysate) required for protein synthesis and an energy source ((1-step Human Copied IVT kit - DNA; Thermoscientific) required for synthesis were supplied to a tube in which the protein expression gel (RCA DNA gel) prepared in stage 4 of Example 1, so that the content in the tube was reacted. Then, the expression of a green fluorescent protein (GFP) was identified with an electrophoretic image analysis system (Geldoc). The results are shown in FIG. 8. As the positive control of FIG. 8, the plasmid used to make the protein expression gel was used instead of the protein expression gel.
2. In order to check the amount of a protein actually produced from the protein expression gel for each reaction time compared to other experimental groups, the amount of the produced protein was measured by real-time PCR, and the results are shown in FIG. 9.
3. Referring to FIG. 8, it is seen that the protein expression gel effectively produces proteins in a cell-free environment. Referring to FIG. 9, it is seen that the protein production continuously increases only in the protein expression gel as time passes. That is, it is seen that a larger amount of protein can be produced more effectively and stably than a solution-based system.

Hereinabove, the applicant has described the preferred embodiments of the present invention, but these embodiments are presented only for illustrative purposes to help implementation of the technical idea of the present invention. Thus, any other changes and modifications should be interpreted as falling within the scope of the present invention as long as the technical idea of the present invention is implemented.

## Claims

1. A method for preparing a cell-free protein expression gel comprising:
a process of preparing a plasmid that preparing a circular double-stranded plasmid which comprises a nucleotide sequence containing genetic information for expressing a target protein;
a process of separating a plasmid that separating the circular double-stranded plasmid into a circular single-stranded plasmids; and
a process of forming a gel that by supplying DNA polymerase to a container which contains the circular single-stranded plasmids saperated from the process of separating a plasmid and then incubating, DNA strand generated from each of the single-stranded plasmid are bound(hybridized) complementally to form the cell-free protein expression gel.

2. The method of claim 1, wherein the process of separating a plasmid is **characterized in that** separating the circular double-stranded plasmid into the circular single-stranded plasmid by breaking a hydrogen bond between the double strand by applying heat to the circular double-stranded plasmid.

3. The method of claim 1, wherein through the process of separating a plasmid, two circular single-stranded plasmids which are complementary to each other are formed from one circular double-stranded plasmid.

4. The method of claim 1, wherein the circular double-stranded plaismid comprises:
a first strand comprising the nucleotide sequence containing genetic information for expressing the target protein; and
a second strand having a nucleotide sequence complementary to the first strand, wherein the first strand and the second strand are bonded complementally.

5. The method of claim 1, wherein the process of forming a gel comprises:
a primer- binding process that binding a primer with the single-stranded plasmid, wherein the primer has a complementary nucleotide sequence to the circular single-stranded plasmid, in order to repeatedly generate a desired DNA from the separated circular single-stranded plasmid;
a polymerase- supplying process that supplying DNA polymerase to the container which contains the circular single-stranded plasmids which are bonded with the primer, after the primer- binding process,; and
a incubating- process that treating the container at a certain temperature for a certain time, after the polymerase supplying process.

6. The method of claim 5, wherein two types of primers which have different nucleotide sequence each other are used in the primer binding process.

7. The method of claim 5,
wherein the incubating process is **characterized in that** long DNA strands are continuously generated from each of the circular single-stranded plasmid, wherein since the long DNA strands have a complementary nucleotide sequence to each other, the long DNA strands are bonded complementally and tangled each other to form cell-free protein expression gel.

8. The method of claim 5, wherein the incubating process is **characterized in that** performed at 35°C or more to 39°C or less, for 36 hours or more to 96 hours or less.

9. The method of claim 1, wherein the cell-free protein expression gel is **characterized in that** the cell-free protein expression gel is capable of producing the desired protein in cell-free environment, not in cell, by reacting with cell lysate.


<110>    University of Seoul Industry Cooperation Foundation

<120>    Method for preparing cell-free protein expression gel using
         plasmid

<130>    PFAHJ-20135

<150>    KR 2019/0130097
<151>    2019-10-18

<160>    4

<170>    KoPatentIn 3.0

<210>    1
<211>    2858
<212>    DNA
<213>    Artificial Sequence

<220>
<223>    top strand of plasmid DNA


<400>    1
cccgtgtaaa acgacggcca gtttatctag tcagcttgat tctagctgat cgtggaccgg        60

aaggtgagcc agtgagttga ttgcagtcca gttacgctgg agtctgaggc tcgtcctgaa       120

tgatatgcga ccgccggagg gttgcgtttg agacgggcga cagatcgaca ctgctcgatc       180

cgctcgcacc taatacgact cactataggg atgccaccat ggatggtgag caagggcgag       240

gagctgttca ccggggtggt gcccatcctg gtcgagctgg acggcgacgt aaacggccac       300

aagttcagcg tgtccggcga gggcgagggc gatgccacct acggcaagct gaccctgaag       360

ttcatctgca ccaccggcaa gctgcccgtg ccctggccca ccctcgtgac caccctgacc       420

tacggcgtgc agtgcttcag ccgctacccc gaccacatga agcagcacga cttcttcaag       480

tccgccatgc ccgaaggcta cgtccaggag cgcaccatct tcttcaagga cgacggcaac       540

tacaagaccc gcgccgaggt gaagttcgag ggcgacaccc tggtgaaccg catcgagctg       600

aagggcatcg acttcaagga ggacggcaac atcctggggc acaagctgga gtacaactac       660

aacagccaca acgtctatat catggccgac aagcagaaga acggcatcaa ggtgaacttc       720

aagatccgcc acaacatcga ggacggcagc gtgcagctcg ccgaccacta ccagcagaac       780

acccccatcg gcgacggccc cgtgctgctg cccgacaacc actacctgag cacccagtcc       840

gccctgagca aagaccccaa cgagaagcgc gatcacatgg tcctgctgga gttcgtgacc       900

gccgccggga tcactctcgg catggacgag ctgtacaagt aaggatcgac gagagcagcg       960

cgactggatc agttctggac gagcgagctg tcgtccgacc cgtgatctta cggcattata      1020

cgtatgatcg gtccacgatc agctagatta tctagtcagc ttgatgtcat agctgtttcc      1080

tgaggctcaa tactgaccat ttaaatcata cctgacctcc atagcagaaa gtcaaaagcc      1140

tccgaccgga ggcttttgac ttgatcggca cgtaagaggt tccaactttc accataatga      1200

aataagatca ctaccgggcg tattttttga gttatcgaga ttttcaggag ctaaggaagc 1260

taaaatgagt attcaacatt tccgtgtcgc ccttattccc ttttttgcgg cattttgcct 1320

tcctgttttt gctcacccag aaacgctggt gaaagtaaaa gatgctgaag atcagttggg 1380

tgcacgagtg ggttacatcg aactggatct caacagcggt aagatccttg agagtttacg 1440

ccccgaagaa cgttttccaa tgatgagcac ttttaaagtt ctgctatgtg gcgcggtatt 1500

atcccgtatt gacgccgggc aagagcaact cggtcgccgc atacactatt ctcagaatga 1560

cttggttgag tactcaccag tcacagaaaa gcatctcacg gatggcatga cagtaagaga 1620

attatgcagt gctgccataa ccatgagtga taacactgcg gccaacttac ttctggcaac 1680

gatcggagga ccgaaggagc taaccgcttt tttgcacaac atgggggatc atgtaactcg 1740

ccttgatcgt tgggaaccgg agctgaatga agccatacca aacgacgagc gtgacaccac 1800

gatgcctgta gcaatggcaa caacgttgcg caaactatta actggcgaac tacttactct 1860

agcttcccgg caacaattaa tagactggat ggaggcggat aaagttgcag gatcacttct 1920

gcgctcggcc ctcccggctg gctggtttat tgctgataaa tctggagccg gtgagcgtgg 1980

gtctcgcggt atcattgcag cactggggcc agatggtaag ccctcccgca tcgtagttat 2040

ctacacgacg gggagtcagg caactatgga tgaacgaaat agacagatcg ctgagatagg 2100

tgcctcactg attaagcatt ggtaatgagg gcccaaatgt aatcacctgg ctcaccttcg 2160

ggtgggcctt tcttgaggac ctaaatgtaa tcacctggct caccttcggg tgggcctttc 2220

tgcgttgctg gcgtttttcc ataggctccg cccccctgac gagcatcaca aaaatcgatg 2280

ctcaagtcag aggtggcgaa acccgacagg actataaaga taccaggcgt ttccccctgg 2340

aagctccctc gtgcgctctc ctgttccgac cctgccgctt accggatacc tgtccgcctt 2400

tctcccttcg ggaagcgtgg cgctttctca tagctcacgc tgtaggtatc tcagttcggt 2460

gtaggtcgtt cgctccaagc tgggctgtgt gcacgaaccc cccgttcagc ccgaccgctg 2520

cgccttatcc ggtaactatc gtcttgagtc caacccggta agacacgact tatcgccact 2580

ggcagcagcc actggtaaca ggattagcag agcgaggtat gtaggcggtg ctacagagtt 2640

cttgaagtgg tggcctaact acggctacac tagaagaaca gtatttggta tctgcgctct 2700

gctgaagcca gttacctcgg aaaaagagtt ggtagctctt gatccggcaa acaaaccacc 2760

gctggtagcg gtggtttttt tgtttgcaag cagcagatta cgcgcagaaa aaaaggatct 2820

caagaagatc ctttgatttt ctaccgaaga aaggccca 2858

<210> 2
<211> 2858
<212> DNA
<213> Artificial Sequence

<220>
<223>    bottom strand of plasmid DNA

<400>    2
gggcacattt tgctgccggt caaatagatc agtcgaacta agatcgacta gcacctggcc      60

ttccactcgg tcactcaact aacgtcaggt caatgcgacc tcagactccg agcaggactt     120

actatacgct ggcggcctcc caacgcaaac tctgcccgct gtctagctgt gacgagctag     180

gcgagcgtgg attatgctga gtgatatccc tacggtggta cctaccactc gttcccgctc     240

ctcgacaagt ggccccacca cgggtaggac cagctcgacc tgccgctgca tttgccggtg     300

ttcaagtcgc acaggccgct cccgctcccg ctacggtgga tgccgttcga ctgggacttc     360

aagtagacgt ggtggccgtt cgacgggcac gggaccgggt gggagcactg gtgggactgg     420

atgccgcacg tcacgaagtc ggcgatgggg ctggtgtact tcgtcgtgct gaagaagttc     480

aggcggtacg ggcttccgat gcaggtcctc gcgtggtaga agaagttcct gctgccgttg     540

atgttctggg cgcggctcca cttcaagctc ccgctgtggg accacttggc gtagctcgac     600

ttcccgtagc tgaagttcct cctgccgttg taggaccccg tgttcgacct catgttgatg     660

ttgtcggtgt tgcagatata gtaccggctg ttcgtcttct tgccgtagtt ccacttgaag     720

ttctaggcgg tgttgtagct cctgccgtcg cacgtcgagc ggctggtgat ggtcgtcttg     780

tgggggtagc cgctgccggg gcacgacgac gggctgttgg tgatggactc gtgggtcagg     840

cgggactcgt ttctggggtt gctcttcgcg ctagtgtacc aggacgacct caagcactgg     900

cggcggccct agtgagagcc gtacctgctc gacatgttca ttcctagctg ctctcgtcgc     960

gctgacctag tcaagacctg ctcgctcgac agcaggctgg gcactagaat gccgtaatat    1020

gcatactagc caggtgctag tcgatctaat agatcagtcg aactacagta tcgacaaagg    1080

actccgagtt atgactggta aatttagtat ggactggagg tatcgtcttt cagttttcgg    1140

aggctggcct ccgaaaactg aactagccgt gcattctcca aggttgaaag tggtattact    1200

ttattctagt gatggcccgc ataaaaaact caatagctct aaaagtcctc gattccttcg    1260

attttactca taagttgtaa aggcacagcg ggaataaggg aaaaaacgcc gtaaaacgga    1320

aggacaaaaa cgagtgggtc tttgcgacca ctttcatttt ctacgacttc tagtcaaccc    1380

acgtgctcac ccaatgtagc ttgacctaga gttgtcgcca ttctaggaac tctcaaatgc    1440

ggggcttctt gcaaaaggtt actactcgtg aaaatttcaa gacgatacac cgcgccataa    1500

tagggcataa ctgcggcccg ttctcgttga gccagcggcg tatgtgataa gagtcttact    1560

gaaccaactc atgagtggtc agtgtctttt cgtagagtgc ctaccgtact gtcattctct    1620

taatacgtca cgacggtatt ggtactcact attgtgacgc cggttgaatg aagaccgttg    1680

ctagcctcct ggcttcctcg attggcgaaa aaacgtgttg taccccctag tacattgagc    1740

ggaactagca acccttggcc tcgacttact tcggtatggt ttgctgctcg cactgtggtg 1800

ctacggacat cgttaccgtt gttgcaacgc gtttgataat tgaccgcttg atgaatgaga 1860

tcgaagggcc gttgttaatt atctgaccta cctccgccta tttcaacgtc ctagtgaaga 1920

cgcgagccgg gagggccgac cgaccaaata acgactattt agacctcggc cactcgcacc 1980

cagagcgcca tagtaacgtc gtgaccccgg tctaccattc gggagggcgt agcatcaata 2040

gatgtgctgc ccctcagtcc gttgatacct acttgcttta tctgtctagc gactctatcc 2100

acggagtgac taattcgtaa ccattactcc cgggtttaca ttagtggacc gagtggaagc 2160

ccacccggaa agaactcctg gatttacatt agtggaccga gtggaagccc acccggaaag 2220

acgcaacgac cgcaaaaagg tatccgaggc ggggggactg ctcgtagtgt ttttagctac 2280

gagttcagtc tccaccgctt tgggctgtcc tgatatttct atggtccgca aagggggacc 2340

ttcgagggag cacgcgagag gacaaggctg ggacggcgaa tggcctatgg acaggcggaa 2400

agagggaagc ccttcgcacc gcgaaagagt atcgagtgcg acatccatag agtcaagcca 2460

catccagcaa gcgaggttcg acccgacaca cgtgcttggg gggcaagtcg ggctggcgac 2520

gcggaatagg ccattgatag cagaactcag gttgggccat tctgtgctga atagcggtga 2580

ccgtcgtcgg tgaccattgt cctaatcgtc tcgctccata catccgccac gatgtctcaa 2640

gaacttcacc accggattga tgccgatgtg atcttcttgt cataaaccat agacgcgaga 2700

cgacttcggt caatggagcc tttttctcaa ccatcgagaa ctaggccgtt tgtttggtgg 2760

cgaccatcgc caccaaaaaa acaaacgttc gtcgtctaat gcgcgtcttt ttttcctaga 2820

gttcttctag gaaactaaaa gatggcttct ttccgggt 2858

<210> 3
<211> 20
<212> DNA
<213> Artificial Sequence

<220>
<223> primer for circular DNA

<400> 3
aagtcgtgct gcttcatgtg 20

<210> 4
<211> 22
<212> DNA
<213> Artificial Sequence

<220>
<223> primer for circular DNA

<400> 4
taatacgact cactataggg at 22